Europäisches Patentamt

European Patent Office

Office européen des brevets

(11) Veröffentlichungsnummer: **0 166 969**
**A2**

(12) # EUROPÄISCHE PATENTANMELDUNG

(21) Anmeldenummer: 85106655.5

(22) Anmeldetag: 30.05.85

(51) Int. Cl.⁴: **A 61 K 31/225**
C 07 C 69/34, C 07 C 69/604
C 07 C 69/704

(30) Priorität: 01.06.84 DE 3420390

(43) Veröffentlichungstag der Anmeldung:
08.01.86 Patentblatt 86/2

(84) Benannte Vertragsstaaten:
BE CH DE FR GB LI NL

(71) Anmelder: Medi-Pharma Vertriebsgesellschaft mbH
Sachsenring 37-47
D-5000 Köln 1(DE)

(72) Erfinder: Fink-Gremmels, Johanna, Dr.
Am Ortsfeld 76
D-3004 Isernhagen NB(DE)

(72) Erfinder: Arnold, Alfred, Dr.
Hermann-Hesse Strasse 9
D-5047 Wesseling(DE)

(72) Erfinder: Otte, Burchard, Dr.
Balsaminenweg 4
D-5000 Köln 71(DE)

(74) Vertreter: Werner, Hans-Karsten, Dr. et al,
Deichmannhaus am Hauptbahnhof
D-5000 Köln 1(DE)

(54) Hämostypticum.

(57) Ein neues Hämostypticum enthält als Wirkstoff Tri-n-butylcitrat (TBC) und/oder Trihexylcitrat, Aconit-tributylester, Tricarballylsäure-tributylester. Es kann insbesondere zusammen mit Lösungsmitteln, Lösungsvermittlern und Dispersionsmitteln zu verschiendenen Applikationsformen verarbeitet werden.

EP 0 166 969 A2

Gegenstand der vorliegenden Erfindung ist ein neues
Hämostypticum, welches in verschiedenen Applikationsformen zur Anwendung kommen kann.

Unter einem Hämostypticum versteht man Mittel zur Blutstillung, die sowohl die Blutungszeit als auch den
Blutverlust bei mechanischen Verletzungen herabsetzen.
Die Blutgerinnung ist ein in den letzten Jahren intensiv untersuchter komplexer Vorgang, der sich in verschiedenster Weise beeinflussen läßt. Die Blutstillung
und der Blutverlust hängen wiederum in erster Linie vom
Umfang und der Geschwindigkeit der Blutgerinnung ab,
jedoch spielen hier auch noch weitere Faktoren mit
hinein, die bisher noch nicht ausreichend untersucht
und geklärt werden konnten. Eine besondere Schwierigkeit bei der Untersuchung von Hämostyptica ist die Erarbeitung von zuverlässigen und reproduzierbaren Versuchsbedingungen am Menschen und am Tier. Eine umfassende Zusammenstellung der bisher bekannten Methoden
sowie die Beschreibung einer verbesserten Methode an
der Ratte wird beschrieben in der Dissertation von Ennen 1981, am Institut für Pharmakologie, Toxikologie
und Pharmazie der Tierärztlichen Hochschule Hannover
unter Leitung von Prof. Dr. K. Kaemmerer (Methodenbeitrag zur Bestimmung von Blutungszeit und Blutverlust
bei Leberparenchymblutungen kleiner Versuchstiere). Im
Rahmen dieser Untersuchungen wurden auch verschiedene
bekannte Hämostasesysteme vergleichend untersucht. Zu
den untersuchten Produkten gehörte auch das Hämostypticum Revici, welches von der Anmelderin seit Jahren
vertrieben wird. Ennen berichtet in der Dissertation,
daß das Hämostypticum Revici bei oraler und intravenöser Verabreichung eine signifikante Verkürzung der Blutungszeit der Rattenleber bewirkt. Das Hämostypticum Revici enthält neben geringen Mengen von Zitronensäure, Wasserstoffperoxid und n-Butanol. Da bei der Anwendung des Hämostypticums Revici immer wieder schwankende und nicht immer reproduzierbare Ergebnisse beobachtet wurden, wurden eingehende Untersuchungen über die Wirksamkeit aller drei Komponenten und Gemische derselben auf ihre Wirksamkeit hin untersucht. Dabei dienten als Modelle in erster Linie die bereits von Ennen entwickelten Tiermodelle der Ratten sowie ähnliche Versuche an der Schweineleber. Die Untersuchungen wurden zusätzlich ausgedehnt auf mögliche Metaboliten und Zersetzungsprodukte der Zitronensäure, nämlich Cis-Aconitsäure, Acetondicarbonsäure und Itakonsäure. Hierbei zeigte sich, daß Zitronensäure und ihre Zersetzungsprodukte praktisch wirkungslos sind, während n-Butanol und $H_2O_2$ für sich und in Kombination einen gewissen Effekt zeigen. Am stärksten wirksam ist jedoch das Gemisch aus den drei Komponenten Zitronensäure, $H_2O_2$ und n-Butanol, nämlich das Hämostypticum Revici.

Weitere eingehende Untersuchungen führten zu dem überraschenden Ergebnis, daß Tri-n-butylcitrat eine sehr starke und reproduzierbare Wirksamkeit aufweist, obwohl dieses Umsetzungsprodukt aus Zitronensäure und n-Butanol im Hämostypticum Revici nicht nachgewiesen oder identifiziert werden konnte.

Tri-n-butylcitrat (TBC) ist eine bekannte Substanz, die bisher nur verwendet wurde als Weichmacher und Lösungsmittel für Nitrocelluloselacke, in Poliermitteln, Tinten und ähnlichen Zubereitungen sowie als Antischaummittel. Es handelt sich um eine in Wasser unlösliche Flüssigkeit vom Schmelzpunkt -20°C, die in anorganischen Lösungsmitteln gut löslich ist, vgl. Merck Index, 10. Auflage, 1983 Nr. 1537.

Weiterhin wurde jetzt eine große Anzahl weiterer Ester mehrwertiger organischer Säuren mit niederen Alkoholen auf hämostyptische Wirkung getestet. Dabei wurde gefunden, daß die meisten dieser Substanzen unwirksam, nur sehr schwach wirksam und teilweise sogar negativ wirksam sind, d.h. sogar gerinnungshemmende Wirkung aufweisen. Eine durchaus interessante und für die Praxis brauchbare Wirkung bei niedriger Toxizität wurde gefunden für Trihexylcitrat, Aconit-tributylester und Tricarballylsäure-tributylester. Diese drei Substanzen sind bisher in der Literatur nicht beschrieben, können jedoch nach an sich bekannten Methoden zur Herstellung solcher Ester hergestellt werden.

Gegenstand der vorliegenden Erfindung ist somit ein neues Hämostypticum enthaltend als Wirkstoff Tri-n-butylcitrat (TBC) und/oder Trihexylcitrat, Aconit-tributylester, Tricarballysäure-tributylester.

Ein weiterer Gegenstand der Erfindung ist die Verwendung von Tri-n-butylcitrat und/oder Trihexylcitrat, Aconit-tributylester und Tricarballylsäure-tributylester als Hämostypticum bzw. zur Herstellung von Hämostyptica. Schließlich sind Gegenstand der vorliegenden Erfindung die drei neuen Ester Trihexylcitrat, Aconit-tributylester und Tricarballylsäure-tributylester.

Diese Ester sind sowohl oral als auch intravenös wirksam und können daher in den verschiedensten Applikationsformen angewendet werden. Eine besondere Schwierigkeit bei der Anwendung besteht darin, daß sie in Wasser extrem schwer löslich sind und Lösungen in organischen Lösungsmitteln bei Zusatz von Wasser rasch zur Phasentrennung führen. Insbesondere bei der oralen
Applikation kann die schlechte Wasserlöslichkeit dazu
führen, daß die Resorption langsam und unvollständig
ist und daher höhere Dosen angewendet werden müssen.
Wegen der geringen Toxizität kann dies jedoch gefahrlos
verantwortet werden.

Bei oralen Applikationsformen ist weiterhin zu beachten, daß TBC und die neuen Ester nachhaltig ätzend und
bitter schmecken und daher die Herstellung von gut
trinkbaren Lösungen Schwierigkeiten bereitet.

Sofern das Hämostypticum zur Vorbereitung von chirurgischen Eingriffen prophylaktisch gegeben werden soll,
kommen daher für die orale Applikation auch Kapseln und
Dragees in Frage. Für die rasche Behandlung akuter Blutungen kommen hingegen vorzugsweise trinkbare Lösungen
in Frage, die den Wirkstoff rasch resorbierbar zur Verfügung stellen.

Bei Injektionslösungen ist es möglich, geeignete organische Lösungsmittel zu verwenden, da bei der Injektion
und Vermischung mit Blut zwar ebenfalls eine Phasentrennung auftritt, der Wirkstoff jedoch in fein disperser Form bis zur Wunde transportiert werden kann.

Als weitere Applikationsform kommen auch Suppositorien
in Frage, aus denen der Wirkstoff relativ rasch resorbiert und in die Blutbahn aufgenommen werden kann.

Das erfindungsgemäße Hämostypticum enthält somit in der
Praxis außer dem Wirkstoff vorzugsweise ein Lösungsmittel, Lösungsvermittler und/oder Dispersionsmittel.

Die Dosis kann im allgemeinen 5 bis 500 mg TBC und/oder einen oder mehrere der drei neuen Ester enthalten. Insbesondere bei oralen Applikationsformen sind Dosierungen von 30 bis 300 mg bevorzugt. Bei intravenöser Applikation sind Dosierungen von 10 bis 100 mg ausreichend, vorzugsweise werden 20 bis 50 mg in einem Lösungsmittel injiziert. Bei Suppositorien sollte die applizierte Menge ebenfalls vorzugsweise zwischen 30 und 300 mg liegen.

In allen Fällen sollte eine Unterdosierung vermieden werden, da diese zu einem unzureichenden Effekt führt. Überdosierungen hingegen sind wegen der geringen Toxizität und guten Verträglichkeit des TBC und der neuen drei Ester ungefährlich. Sie garantieren hingegen mit Sicherheit, daß auch bei schwankender Resorption oder Transportverlusten in der Blutbahn ausreichende Mengen des Wirkstoffes in die frische Wunde gelangen.

Nach den bisher vorliegenden Untersuchungen ist TBC im Hämostypticum Revici nicht vorhanden und nicht nachweisbar. Sollten spätere Untersuchungen zeigen, daß auch im Hämostypticum Revici Anteile der Zitronensäure sich mit dem n-Butanol zum TBC umgesetzt haben, so ist doch die Wirksamkeit des Hämostypticums Revici mit Sicherheit nicht auf diese geringen Mengen TBC zurückzuführen, da selbst bei vollständiger Umsetzung der Zitronensäure mit den überschüssigen n-Butanol sich maximal 5 mg TBC/Dosis hätten bilden können. Diese Menge liegt aber immer noch unterhalb der erfindungsgemäßen erforderlichen Mindestdosis.

Das Hämostypticum Revici enthält insbesondere erhebliche Mengen n-Butanol, welches zwar eine gewisse hämostyptische Wirksamkeit besitzt, jedoch erst in Mengen die nicht allzuweit unterhalb der toxischen Dosen liegen. Bei den Schweineversuchen hat sich beispielsweise gezeigt, daß die im Hämostypticum Revici vorhandene Menge n-Butanol für sich allein schon zu Kreislaufdepressionen führt. Die hämostyptische Wirkung von TBC wiederum ist wesentlich höher als die von n-Butanol. Die Wirksamkeit kann daher nicht dadurch erklärt werden, daß das TBC im Körper zu Zitronensäure und n-Butanol gespalten wird.

Ein weiterer Hinweis darauf, daß tatsächlich das TBC die hämostyptische Wirkung aufweist, besteht darin, daß die $LD_{50}$ von n-Butanol bei der Ratte 4,36 g/kg beträgt (vgl. Merck Index, 10. Auflage Nr. 1513), während eigene Versuche mit Ratten gezeigt haben, daß die $LD_{50}$ von TBC 20,8 g/kg ist. Bei intravenöser Applikation (Infusion) trat der spontane Exitus bei der Ratte nach 19,4 ml/kg auf, während unter gleichen Bedingungen bereits nach Infusion von 9,9 ml Hämostypticum Revici der Exitus erfolgt. Hierbei ist zu beachten, daß bei der Ratte als maximale Volumenbelastung 20 ml/kg gelten.

Weitere Untersuchungen von TBC an der Ratte haben gezeigt, daß oral weder bei 10 ml/kg noch bei 20 ml/kg entsprechend 10,4 mg/kg bzw. 20,8 mg/kg keinerlei akute oder klinische manifeste Veränderungen beobachtet wurden. TBC ist somit als ausgesprochen sichere und ungefährliche Substanz angesehen.

In den nachfolgenden Beispielen sind einige typische Zubereitungen des neuen Hämostypticums beschrieben.

B e i s p i e l   1

Ein Teil Tri-n-butylcitrat (TBC), (Merck, Darmstadt Nr. 820350, Chargen-Nr. 3193097) wurden mit 8 Teilen aus Polyoxyethylensorbitansäureesters (Arlatone 980) vermischt. Durch 2 bis 4 Gew.-% Saccharin-NA und 1 bis 2%
Zitronensäure wurde der bittere Geschmack teilweise
überdeckt und der pH-Wert der Lösung auf ca. 2,5 eingestellt. Dieses Gemisch ist in Wasser löslich und kann
ohne weiteres oral appliziert werden. Wiederholungen
des Versuches mit anderen Arlatonen und anderen
Mischungsverhältnissen von Arlatonen und TBC haben ergeben, daß auch diese wasserausreichend löslich und
deshalb als orale Darreichungsform geeignet sind.

### B e i s p i e l  2

100 mg TBC wurden mit 400 mg Neutralölen wie Sojaöl,
Sonnenblumenöl oder mit Migliol vermischt und in Weichgelantinekapseln eingebracht. Diese Kapseln sind sehr
gut einnehmbar, setzen jedoch den Wirkstoff langsamer
frei als die Lösungen gemäß Beispiel 1.

### B e i s p i e l  3

0,2 ml TBC wurden in 0,8 ml Tetraglycol gelöst. Dieser
Lösung kann bis zu 0,2 ml Wasser zugegeben werden, ohne
daß sich die Lösung trübt. Das Gemisch aus 20 Vol-% TBC
und 80 Vol-% Tetraglykol kann intravenös injiziert werden und ist gut verträglich.

Eine ebenfalls gut verträgliche Injektionslösung erhält
man aus 0,2 ml TBC, 0,6 ml Tetraglycol und 0,2 ml Ethanol. Auch diese Lösung ist klar und kann mit etwas Wasser versetzt werden, ehe sie sich trübt.

### B e i s p i e l  4

2,4 g TBC, 0,3 g pyrogene Kieselsäure (Aerosil 200) und 13,5 g übliche Suppositorienmasse (Stadimol) wurden miteinander vermischt und zu Suppositorien von ca. 2 g verarbeitet. Jedes Suppositorium enthielt somit ca. 300 mg TBC.

Beispiel 5

Analog der für Tributylcitrat bekannten Methode wurde Zitronensäure mit n-Hexanol vollständig verestert und ergab eine Flüssigkeit, die chromatographisch als praktisch reines Trihexylcitrat identifiziert werden konnte. Dieses Öl konnte in gleicher Weise wie in den Beispielen 1 bis 4 beschrieben in galenische Applikationsformen überführt werden.

Beispiel 6

Aconitsäure und Tricarballylsäure wurden vollständig mit n-Butanaol verestert und ergaben Öle, die chromatographisch als praktisch reine Aconit-butylester bzw. Tricarballylsäure-tributylester identifiziert werden konnten. Die Substanzen wurden in analoger Weise zu den Beispielen 1 bis 4 in galenische Applikationsformen überführt.

Beispiel 7

Die Wirksamkeit der Substanzen wurde sowohl oral als auch IV getestet mit Standardlösungen suspendiert in 0,9%-iger Kochsalzlösung bei Ratten nach Ennen (1981). Gemessen wurden Blutungszeit und Blutverlust in % der Abnahme. Tributylcitrat ergab eine Abnahme der Blutungszeit von 27% und eine Abnahme des Blutverlustes von 44%. Die entsprechenden Werte für Trihexylcitrat lagen bei 20 bzw. 24%. Die Werte für Aconit-tributyl-

C166969

ester bei 22 bzw. 16% und für Tricarballylsäure-tributylester bei 29 bzw. 8%.

Patentansprüche

1. Hämostypticum enthaltend als Wirkstoff Tri-n-butylcitrat (TBC) und/oder Trihexylcitrat, Aconit-tributylester, Tricarballylsäure-tributylester.

2. Hämostypticum gemäß Anspruch 1, dadurch gekennzeichnet,
   daß der Wirkstoff in Mengen von 5 bis 500 mg/Dosis vorliegt.

3. Hämostypticum gemäß Anspruch 1 oder 2, dadurch gekennzeichnet, daß es Lösungsmittel, Lösungsvermittler
   und/oder Dispersionsmittel enthält.

4. Hämostypticum gemäß Anspruch 3, dadurch gekennzeichnet,
   daß es als Lösungsmittler und/oder Dispersionsmittel
   Polyoxyethylensorbitansäureester enthält.

5. Verwendung von TBC und/oder Trihexylcitrat, Aconit-tributylester, Tricarballylsäure-tributylester als Hämostypticum.

6. Verwendung von TBC und/oder Trihexylcitrat, Aconit-tributylester, Tricarballylsäure-tributylester zur Herstellung von Hämostyptica.

7. Trihexylcitrat, Aconit- tributylester und Tricarballyl-
   säure-tributylester.